# EUROPEAN PATENT APPLICATION

(11) **EP 4 268 890 A1**
(43) Date of publication of application: **01.11.2023**
(21) Application number: 22170559.3
(22) Date of filing: 28.04.2022
(51) Int. Cl.: A61P 3/10, C07K 7/56, C07K 7/64

(54) **METHYLGLYOXAL-SCAVENGING CYCLIC PEPTIDES AND THEIR USE FOR THE PREVENTION AND TREATMENT OF DISEASES ASSOCIATED WITH ELEVATED METHYLGLYOXAL LEVELS**

(71) Applicant: Universität Heidelberg, 69117 Heidelberg (DE)
(72) Inventor: Brings, Sebastian, 69181 Leimen (DE); Mier, Walter, 64625 Bensheim (DE); Nawroth, Peter P., 69181 Leimen (DE)
(74) Representative: Grahn, Sibylla Maria

(57) **Abstract**

The present invention relates to cyclic peptide compounds which inhibit or antagonize the binding of methylglyoxal (MG) and/or other reactive carbonyl species (RCS) to an arginine- or lysine- containing protein. Preferred scavenger compounds are said cyclic peptides comprising at least two lysines, at least one amino acid with an acidic side chain, at least one Dap and a hydrophobic modification, and pharmaceutical compositions thereof. The present invention furthermore relates to the use of the cyclic peptides as scavenger or antagonists of methylglyoxal and/or related reactive carbonyl species (RCS). The present invention furthermore relates to the use of the cyclic peptides for the prevention and/or treatment of a disease caused by or associated with methylglyoxal (MG) and/or reactive carbonyl species (RCS), in particular caused by or associated with elevated MG levels, such as diabetes and its associated complications, cardiovascular diseases and obesity.

## Description

The present invention relates to cyclic peptide compounds which inhibit or antagonize the binding of methylglyoxal (MG) and/or other reactive carbonyl species (RCS) to an arginine- or lysine- containing protein. Preferred scavenger compounds are said cyclic peptides comprising at least two lysines, at least one amino acid with an acidic side chain, at least one Dap and a hydrophobic modification, and pharmaceutical compositions thereof. The present invention furthermore relates to the use of the cyclic peptides as scavenger or antagonists of methylglyoxal and/or related reactive carbonyl species (RCS). The present invention furthermore relates to the use of the cyclic peptides for the prevention and/or treatment of a disease caused by or associated with methylglyoxal (MG) and/or reactive carbonyl species (RCS), in particular caused by or associated with elevated MG levels, such as diabetes and its associated complications, cardiovascular diseases and obesity.

### BACKGROUND OF THE INVENTION

Diabetes is defined by high blood glucose levels. Despite the high efficacy of the modern standard therapies, diabetic patients develop serious complications in part caused by reactive dicarbonyl compounds. These dicarbonyls form protein adducts termed advanced glycation end-products (AGEs), one of the factors for the development of diabetic complications (Thornalley *et al.,* 2003; Rabbani *et al.,* 2014). Methylglyoxal (MG) represents the most abundant reactive dicarbonyl compound in the plasma of diabetic patients (Fleming *et al*., 2012). The formation of MG derived protein adducts is associated with diabetic nephropathy, diabetic retinopathy, diabetic neuropathy and endothelial dysfunction (Beisswenger *et al.* 2005, Beisswenger *et al.* 2013, Genuth *et al.* 2015, Maessen *et al.* 2015, Giacco *et al.* 2014). Furthermore, MG is involved in the development of cardiovascular complications (Schalkwijk *et al.* 1998, Rabbani *et al.* 2010, Rabbani *et al.* 2011). There it contributes to atherosclerosis via modification of low density lipoprotein, increasing its atherogenicity while affecting binding to- and thus clearance via the LDL receptor. With regard to diabetic neuropathy we have previously shown that MG is causative of hyperalgesia, an increased sensitivity towards pain, associated with diabetic neuropathy (Bierhaus *et al.,* 2012). Additional support for a deleterious effect of MG on neurons during development comes from a study of maternal diabetes in mice (Yang, Cancino *et al.* 2016). The MG detoxifying enzyme glyoxalase 1 (Glo1) is also decreased in adipose tissue while overexpression of Glo1 suppresses weight gain linking MG to adiposity (Rabbani and Thornalley 2015).

Additional diseases where an pathogenic effect of MG is emerging and where said compound may be of therapeutic potential are Alzheimer's Disease (Hensley *et al.* 1995, Aksenov *et al.* 2000, Conrad *et al.* 2000, Aksenov *et al.* 2001, Butterfield and Lauderback 2002, Castegna *et al.* 2002, Choi *et al.* 2002, Munch *et al.* 2003, Ahmed *et al.* 2005, Chen *et al.* 2007), amyotrophic lateral sclerosis (Shinpo *et al.* 2000, Ferrante *et al.* 1997), cataractogenesis (Boscia *et al.* 2000, Shamsi *et al.* 1998), chronic renal failure and chronic or acute Uraemia (Miyata *et al.* 1999, Himmelfarb *et al.* 2000, Himmelfarb and McMonagle 2001, Lim *et al.* 2002, Agalou *et al.* 2003, Lapolla *et al.* 2005, Rabbani *et al.* 2007, Muller-Krebs *et al.* 2008, Nakayama *et al.* 2008), cystic fibrosis (McGrath *et al.* 1999, Range *et al.* 1999), dementia with Lewy bodies (Lyras *et al.* 1998), ischaemia-reperfusion (Pantke *et al.* 1999), pre-eclempsia (Zusterzeel *et al.* 2001), psoriasis (Dimon-Gadal *et al.* 2000), rheumatoid arthritis and juvenile chronic arthritis (Mantle *et al.* 1999, Renke *et al.* 2000), severe sepsis (Winterbourn *et al.* 2000, Abu-Zidan *et al.* 2002), systemic amyloidosis (Miyata *et al.* 2000) and Parkinson's Disease (Floor and Wetzel 1998).

Thus, therapeutic lowering of MG levels is a promising approach to treat diabetic complications in particular diabetic neuropathy as well as other diseases associated with deregulation of Glo1 and/or increased MG levels. Consequently, numerous small molecule scavengers of MG have been developed. However, none of these compounds has been proven successful in clinical trials due to side effects or lack of efficacy (Forbes *et al.,* 2013; Maessen *et al.,* 2015; Engelen *et al.,* 2013).

The scavenging reaction is a comparably slow process, therefore, the ideal scavenger has to have a long circulation time, combined with a reactivity which is specific to avoid aberrant activity (Lo *et al*., 1994). It was shown that MG causes endothelial dysfunction similar to that induced by high glucose. MG scavengers (aminogunidine, N-acetyl cysteine) have potential to prevent endothelial dysfunction induced by MG and high glucose concentrations (Dhar *et al.,* 2012). Brings *et al.,* (2017) and WO 2018/087028 A1 describe scavenger peptides which prevent MG induced pain in mice.

The present invention aims to provide improved means and methods for scavenging and/or antagonizing methylglyoxal and/or reactive carbonyl species (RCS), which allow an improved prevention and/or treatment of pain, in particular pain and/or hyperalgesia caused by or associated with methylglyoxal and/or reactive carbonyl species (RCS) as well as the treatment of other diseases associated with excessive MG formation and/or GLO1 deregulation, such as diabetic nephropathy and diabetic retinopathy, endothelial dysfunction and cardiovascular diseases.

### SUMMARY OF THE INVENTION

According to the present invention this object is solved by providing a cyclic peptide which has a length of 3 to 12 amino acids and comprises
(i) at least two **Lys**,
(ii) at least one **amino acid with an acidic side chain**, preferably Glu or Asp,
(ii) at least one **Dap** (2,3-diaminopropanoic acid) attached to the side chain of Lys, and
(iii) at least one hydrophobic modification **H**.

According to the present invention this object is solved by providing the cyclic peptide according to the present invention for use in medicine.

According to the present invention this object is solved by providing the cyclic peptide according to the present invention suitable for use as scavenger of methylglyoxal and/or reactive carbonyl species (RCS).

According to the present invention this object is solved by providing the cyclic peptide according to the present invention suitable as antagonist for binding to arginine-containing protein(s), preferably an arginine containing extracellular or intracellular protein.

According to the present invention this object is solved by providing the cyclic peptide according to the present invention for use in a method of prevention and/or treatment of a disease caused by or associated with methylglyoxal (MG) and/or reactive carbonyl species (RCS), in particular caused by or associated with elevated MG levels.

According to the present invention this object is solved by providing a pharmaceutical composition comprising
at least one cyclic peptide according to the present invention,
optionally a pharmaceutically acceptable carrier and/or excipient.

According to the present invention this object is solved by providing a method for identifying compounds that influence diabetes and its associated complications, in particular diabetes and its associated complications caused by or associated with methylglyoxal and/or reactive carbonyl species (RCS),
comprising
(a) providing a compound to be screened,
(b) providing a cyclic peptide according to the present invention,
(c) determining the effect of the compound to be screened of (a) on the development and/or progression of diabetes and its associated complications by comparing the effect of the compound to be screened of (a) with the effect of the cyclic peptide of (b),
wherein said associated complications comprise diabetic neuropathy, diabetic nephropathy, diabetic retinopathy and endothelial dysfunction.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "3 to 12" should be interpreted to include not only the explicitly recited values of 3 to 12, but also include individual values and sub-ranges within the indicated range. Thus, included in this numerical range are individual values such as 3, 4, 5 .... 10, 11, 12 and sub-ranges such as from 5 to 10, 6 to 8, 7 to 9 etc. This same principle applies to ranges reciting only one numerical value, such as "≤+5". Furthermore, such an interpretation should apply regardless of the breadth of the range or the characteristics being described.

### Methylglyoxal-scavenging cyclic peptide compounds

As outlined above, the present invention provides cyclic peptides.

A cyclic peptide of the present invention has a length of 3 to 12 amino acids.

A cyclic peptide of the present invention comprises (i) at least two lysine (**Lys**).

A cyclic peptide of the present invention comprises (ii) at least one **amino acid with an acidic side chain**, preferably Glu or Asp.

A cyclic peptide of the present invention comprises (iii) at least one **Dap** (2,3-diaminopropanoic acid) attached to the side chain of Lys.

A cyclic peptide of the present invention comprises (iv) at least one hydrophobic modification **H.**

The cyclic peptides of the present invention are preferably cyclized via
(a) head-to-side chain cyclization, preferably the side chain of the C-terminal Glu,
(b) head-to-tail cyclization,
(c) backbone cyclization,
(d) amide condensation of two amino acid side chains (lactam),
(e) thioether formation,
(f) hydrogen bond formation,
   and/or
(g) side chain-to-tail cyclization,

In a preferred embodiment, the cyclic peptides of the present invention are cyclized via head-to-side chain cyclization.

A cyclic peptide of the present invention is characterized by one or more of the following:

### - length

A cyclic peptide of the present invention has a length of 3 to 12 amino acids.

Preferably, the length is 5 to 10 amino acids, more preferably 6 to 8 amino acids.

For example, the length of the cyclic peptides is 6 or 7 amino acids.

### - lysine residues (i)

A cyclic peptide of the present invention comprises at least two lysine (**Lys**).

In a preferred embodiment, the cyclic peptides comprise 2 to 6 **Lys**, more preferably 3 or 4 or 5 Lys.

### - amino acid with an acidic side chain (ii)

A cyclic peptide of the present invention comprises at least one amino acid with an acidic side chain, preferably Glu or Asp.

In a preferred embodiment, the cyclic peptides comprise 1 to 3 amino acids with an acidic side chain, preferably 1 to 3 Glu and/or Asp.

For example, the cyclic peptides comprise 2 Glu,

### - Dap (iii)

A cyclic peptide of the present invention comprises at least one 2,3-diaminopropanoic acid (Dap) attached to the side chain of Lys.

In a preferred embodiment, the cyclic peptides comprise 1 to 4 Dap, more preferably 2 to 4 Dap, even more preferably 2 or 3 Dap.

### - net charge and plasma half-life

Preferably, the cyclic peptides of the present invention can be further characterized by their net charge and/or plasma half-life.

Preferably, the cyclic peptides have a net charge of about ≤+5, such as about +2.

The cyclic peptides can have a net charge of about -1, about +1, about +2, about +3, about +4, about +5.

Preferably, the cyclic peptides have a plasma half-life of up to about one week, such as a range between one day and one week.

Plasma half-life can be determined by LC MS/MS.

### - hydrophobic modification H (iv)

A cyclic peptide of the present invention comprises (iv) at least one hydrophobic modification **H.**

In a preferred embodiment, the hydrophobic modification **H** is an acylation, preferably
an acylation with C10 to C22 fatty acids, more preferably C12 to C22 fatty acids, such as myristoyl (C14), palmitoyl (C16) or stearoyl (C18), even more preferably myristoyl (C14), or
an acylation with C10 to C22 dicarboxylic acids, more preferably C12 to C22 dicarboxylic acids, such as tetradecanedioic acid (C14), hexadecanedioic acid (C16) (Hdd), octadecanedioic acid (C18), even more preferably hexadecanedioic acid (C16),
   or
an acylation with a fatty acid containing phenyl group, such as 4-(*p-*iodophenyl)butyric acid.

Preferably, the hydrophobic modification is attached to the side chain of a lysine residue close to the N-terminus,
optionally via a linker.

In one embodiment, the linker is an amino acid, preferably at least one *D*-Glu, more preferably one *D*-Glu.

In a preferred embodiment the cyclic peptide comprises an amino acid sequence selected from
Lys - Lys - Lys - Lys - Lys - Glu [SEQ ID NO. 1],
Lys - Lys - Glu - Lys - Lys - Glu [SEQ ID NO. 2],
Lys - Lys - Glu - Lys - Glu - Glu [SEQ ID NO. 3], and
Lys - Glu - Lys - Glu - Lys - Lys - Glu [SEQ ID NO. 4].

In a preferred embodiment the cyclic peptide is selected from 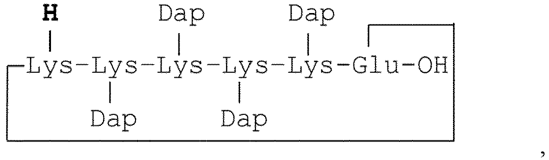 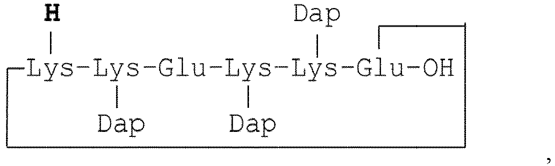 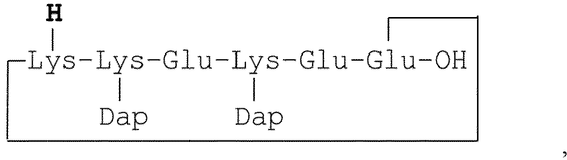 or 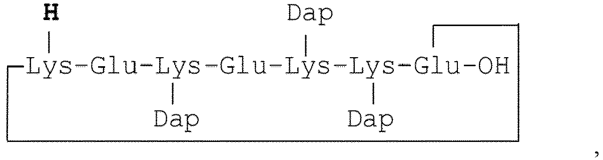 more preferably

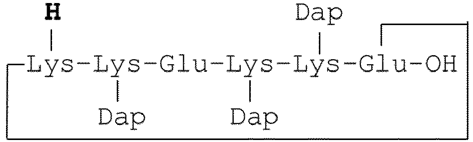

The cyclic peptide according to the present invention is preferably selected from 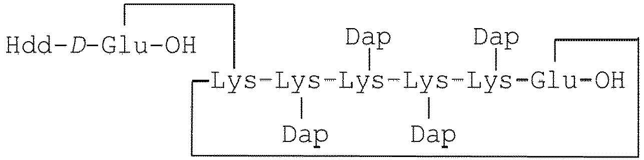 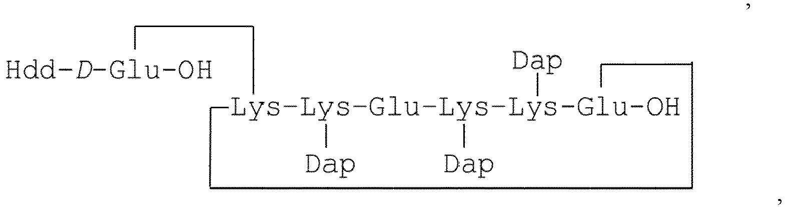 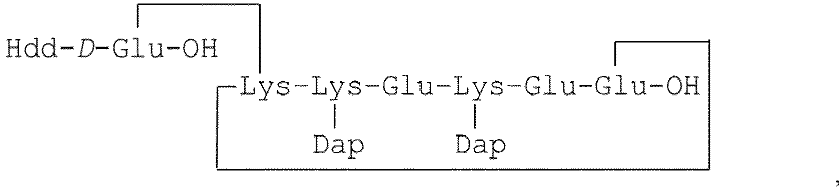 , or 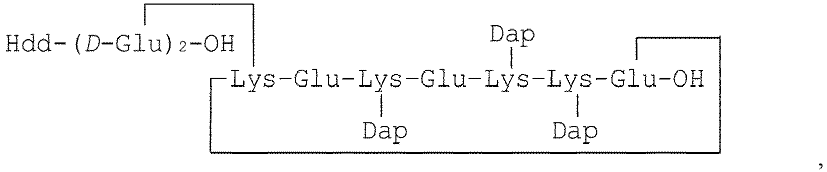 more preferably

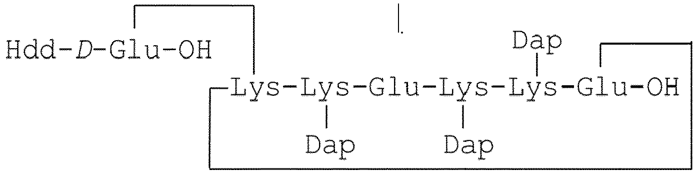

In embodiments of this invention the cyclic peptides can comprise one or more further compounds or moieties, such as tags or labels, e.g. complex agents, fluorescent dyes, radioisotopes and contrast agents.

Said further compound(s) or moieties are preferably covalently attached, such as via a linker, spacer and/or anchor group(s), e.g. a cleavable linker.

In a preferred embodiment, the cyclic peptides according to the present invention inhibit the binding of methylglyoxal (MG) and/or reactive carbonyl species (RCS) to an arginine- or lysine-containing protein.

An arginine- or lysine-containing protein can be an arginine-containing cellular protein, such as a sodium ion channel, e.g. the sodium ion channel Na(v)1.8.

The range of proteins affected includes but is not limited to the renal glomeruli (extra- and intracellular), renal tubules (extra- and intracellular), low density lipoproteins (extracellular in plasma contributing to atherosclerosis) as well as sodium channels, such as the sodium channel Nav 1.8.

A cyclic peptide according to the present invention is preferably capable to
- bind and scavenge methylglyoxal and other RCS *in vivo* and *in vitro,*
   and/or
- antagonize and/or compete with MG and other RCS for binding to proteins, preferably arginine- or lysine-containing extracellular proteins, such as the low density lipoprotein,
   and/or
- prevent the modification of proteins, preferably arginine- or lysine-containing extracellular proteins, such as the low density lipoprotein by MG and other RCS,
   and/or
- antagonize and/or compete with MG and RCS for binding to proteins, preferably arginine- or lysine containing proteins of the glomerulus and tubule of the kidney.
   and/or
- prevent the modification of proteins, preferably arginine- or lysine-containing extracellular proteins, proteins of the glomerulus and tubule of the kidney.
   and/or
- antagonize and/or compete with MG and other RCS for binding to proteins, preferably arginine- or lysine-containing (cellular) proteins, such as the sodium ion channel Na(v)1.8,
   and/or
- prevent the modification of proteins, preferably arginine- or lysine-containing (cellular) proteins, such as the sodium ion channel Na(v)1.8, by MG and other RCS,
   and/or
- inhibit and/or prevent the formation of advanced glycation endproducts (AGEs) by methylglyoxal and other RCS.

The "scavenging potential" of a cyclic peptide as used herein can be viewed as the amount of methylglyoxal which can react with a cyclic peptide of the invention and therefore be the effective amount of methylglyoxal or similarly acting reactive metabolites (such as reactive carbonyl species (RCS)) which are removed from binding to arginine- or lysine-containing (intra- and extracellular) proteins at any situation in which methylglyoxal or similarly acting reactive metabolites (such as reactive carbonyl species (RCS)) are elevated.

### Use of the cyclic peptides as methylglyoxal scavenger and/or antagonist

As outlined above, the present invention provides the cyclic peptides of the present invention as scavengers of methylglyoxal and/or reactive carbonyl species (RCS).

As outlined above, the present invention provides the cyclic peptides of the present invention as antagonists of methylglyoxal for binding to an arginine-containing protein(s), preferably an arginine containing extracellular protein, preferably low density lipoprotein.

As outlined above, an arginine-containing protein includes but is not limited to the renal glomeruli (extra- and intracellular), renal tubules (extra- and intracellular), low density lipoproteins (extracellular in plasma contributing to atherosclerosis) as well as sodium channels, such as the sodium channel Nav 1.8.

Preferably, the cyclic peptides according to the present invention are suitable for use as antagonist for binding to arginine-containing protein(s), preferably an arginine containing extracellular or intracellular protein, such as
- low density lipoprotein,
- Nav1.8,
- glomerular and tubular proteins of the kidney.

In the context of this invention, the term "scavenger" of methylglyoxal and/or "scavenging" methylglyoxal, therefore refers to the potential of the cyclic peptide to prevent the interaction of methylglyoxal (or similarly acting reactive metabolites, such as reactive carbonyl species (RCS)) with protein residues, specifically arginine residues or also lysine residues, in proteins, on and/or in macromolecular protein structures and physiological proteins, as outlined above, *in vitro* as well as *in vivo.*

The cyclic peptides of the invention are suitable as *in vitro* as well as *in vivo* scavengers of methylglyoxal and/or reactive carbonyl species (RCS).

### Pharmaceutical compositions and medical applications

As outlined above, the present invention provides a pharmaceutical composition comprising at least one cyclic peptide as defined herein, and optionally a pharmaceutically acceptable carrier and/or excipient.

The pharmaceutical compositions according to the present invention are very well suited for all the uses and methods described herein.

A "pharmaceutically acceptable carrier or excipient" refers to any vehicle wherein or with which the pharmaceutical compositions according to the invention may be formulated. It includes a saline solution such as phosphate buffer saline. In general, a diluent or carrier is selected based upon the mode and route of administration, and standard pharmaceutical practice.

As outlined above, the present invention further provides the first medical use of the cyclic peptides of this invention.

Thus, the cyclic peptides of this invention are suitable and, thus, provided for the diagnosis, prevention and/or treatment of diseases.

As outlined above, the present invention further provides the cyclic peptides of this invention and/or respective pharmaceutical composition(s) of this invention for the diagnosis, prevention and/or treatment of certain diseases.

In particular, the cyclic peptides of this invention and/or respective pharmaceutical composition(s) of this invention are suitable for the prevention and/or treatment of a disease caused by or associated with methylglyoxal (MG) and/or reactive carbonyl species (RCS).

Said "disease caused by or associated with methylglyoxal (MG) and/or reactive carbonyl species (RCS)" is in particular caused by or associated with elevated MG levels

Said disease is preferably selected from:
- diabetes and its associated complications,
   wherein said associated complications comprise
   diabetic neuropathy (such as pain and/or hyperalgesia),
   diabetic nephropathy (such as albuminuria and/or lowered eGFR),
   diabetic retinopathy, and
   endothelial dysfunction,
- cardiovascular disease,
   in particular atherosclerosis,
- obesity,
- Alzheimers disease, amyotrophic lateral sclerosis, cataractogenesis, chronic renal failure and chronic or acute Uraemia, cystic fibrosis, dementia with Lewy bodies, ischaemia-reperfusion, pre-eclampsia, psoriasis, rheumatoid arthritis and juvenile chronic arthritis, severe sepsis, systemic amyloidosis and Parkinson's disease.

"Pain" (and/or "hyperalgesia") as used herein refers preferably to pain (and/or hyperalgesia) and/or a disease/condition associated with pain and/or hyperalgesia caused by or associated with methylglyoxal and/or reactive carbonyl species (RCS).

The inventors have found that cyclic peptides, which inhibit or antagonize the binding of methylglyoxal (MG) and/or reactive carbonyl species (RCS) to an arginine (or lysine) - containing protein, preferably an arginine (or lysine) -containing cellular protein, such as a sodium ion channel, e.g. the sodium ion channel Na(v)1.8 (the methylglyoxal-scavenging compounds with the characteristics described herein), are suitable for a novel and specific treatment / therapy for pain and/or hyperalgesia, wherein the components causing the pain / hyperalgesia or are associated therewith (namely methylglyoxal (MG) and/or reactive carbonyl species (RCS)) are targeted.

Similar to the prevention of pain via MG scavenging MG can also cause atherosclerosis and as such cardiovascular disease. MG modified low density lipoproteins display an increased atherogenicity while the binding to the LDL receptor is decreased, thus affecting the clearance. An involvement of MG has also been demonstrated in diabetic nephropathy where MG levels are elevated while the modification of proteins with MG are a strong independent predictor of diabetic nephropathy. Furthermore, a decrease in the MG detoxifying enzyme mimicked the development of diabetic nephropathy with increased levels MG-protein adducts, albuminuria and expansion of the mesangial matrix.

In a preferred embodiment the cyclic peptides or the pharmaceutical composition(s) of the invention are used for the manufacture of a medicament for the prevention and/or treatment of atherosclerosis and/or cardiovascular disease caused by or associated with methylglyoxal and/or reactive carbonyl species (RCS).

In a preferred embodiment the cyclic peptides or the pharmaceutical composition(s) of the invention are used for the manufacture of a medicament for the prevention and/or treatment of diabetic nephropathy caused by or associated with methylglyoxal and/or reactive carbonyl species (RCS).

In a preferred embodiment the cyclic peptides or the pharmaceutical composition(s) of the invention are used for the manufacture of a medicament for the prevention and/or treatment of pain and/or hyperalgesia, in particular pain and/or hyperalgesia caused by or associated with methylglyoxal and/or reactive carbonyl species (RCS).

In a preferred embodiment, the cyclic peptides are provided as analgesic.

The pain and/or hyperalgesia to be prevented and/or treated is associated with and/or occurs during a disease, wherein said disease is selected from Alzheimers disease, amyotrophic lateral sclerosis, cataractogenesis, chronic renal failure and chronic and acute Uraemia, cystic fibrosis, dementia with Lewy bodies, diabetes mellitus and its complications (such as nephropathy, neuropathy and retinopathy), ischaemia-reperfusion, pre-eclampsia, psoriasis, rheumatoid arthritis and juvenile chronic arthritis, severe sepsis, systemic amyloidosis, Parkinson's disease, painful bowel disease, chemotherapy induced pain, critical limb ischemia, hypertension, bone pain, tumor pain.

In diabetes mellitus, neuropathy, which is one of three major complications associated with the diseases, is frequently observed with patients exhibiting one or more types of stimulus-evolved pain, including increased responsiveness to noxious stimuli (hyperalgesia) as well as hyper-responsiveness to normally innocuous stimuli (allodynia). The underlying mechanism of persistent pain diabetic patients remains poorly understood and as such there are little or no effective therapeutic treatments which can either delay or prevent the onset of symptoms.

The formation of methylglyoxal and related reactive carbonyl species (RCS) is closely linked to the rate of glycolysis and the presence of glycolytic intermediates. Hence, in conditions where there is increased glycolytic flux and an increased dependence on glycolysis for energy, the rate of methylglyoxal and RCS formation will also be increased. This has been shown to be the case in patients with diabetes mellitus, where complications such as nephropathy, neuropathy and retinopathy have been linked to increases in cellular levels of advanced glycation endproducts (AGEs). While diabetes has been the main area of research, new evidence is now emerging of the pivot role that RCS, in particularly methylglyoxal, plays in the progression and severity of various diseases, such as, but not limited to cardiovascular disease.

For example, methylglyoxal modification of Nav1.8 facilitates nociceptive neuron firing and causes metabolic hyperalgesia:
Small fiber distal polyneuropathy causes persistent hyperalgesia and pain in 10% of people with diabetes. Metabolic hyperalgesia is based on the reactive glycolytic metabolite methylglyoxal (MG). MG exceeding plasma levels of 600nM discriminates between diabetic patients with and without pain, evokes thermal hyperalgesia in mice and induces CGRP release in skin flaps. Cultured sensory neurons treated with MG exhibit intense MG-modifications of arginine residues in the sodium-channel Naᵥ1.8 and increased electrical excitability and membrane resistance. MG effects on action potential generators facilitate firing in nociceptive neurons but inhibit neurons of the autonomic nervous system lacking Naᵥ1.8 expression. The understanding of metabolic driven pain is useful for therapeutic interventions, since an MG binding peptide is able to reduce hyperalgesia in experimental diabetes, thus providing the first pathogenetically based treatment option for painful diabetic neuropathy.

There exists a concept of neuronal dysfunction in certain metabolic diseases. The major insight is the identification (by the inventors) of a key role for local accumulation of the reactive metabolite MG, which by posttranslational modification of the sensory neuronal sodium channel Naᵥ1.8 enhances the excitability and blocks other ion channels including Naᵥ1.7. The concept of metabolic hyperalgesia appears independent of structural changes in the nerve but rather dependent on the molecular interaction of MG with arginine and lysine residues within critical regions of Naᵥ1.8. This observation is compatible with the threshold of about 600 nM MG required to affect neuronal function which was observed in men, mice and peripheral nerve endings. There are several possibilities by which the required MG threshold can be reached. One is the increased metabolic flux of glucose in diabetes through either glycolysis or the pentose phosphate pathway. Under non-diabetic conditions, the incidental amount of MG generated is detoxified by protective enzymes, particularly the glyoxalase system. Other pathological states leading to increased generation of MG are disorders in which acetone and other ketone bodies accumulate, such as uremia, or conditions such as oxidative stress during re-perfusion in which lipid peroxidation occurs.

### Route of administration

Preferably, the route of administration of the cyclic peptides or pharmaceutical compositions of the present invention is selected from subcutaneous, intravenous, oral, nasal, intramuscular, transdermal, inhalative, by suppository.

A preferred embodiment for nasal administration or application is as an inhalant spray, which would be advantageous for cyclic peptide(s), as it would not only allow for faster acting effect, but also limit degradation which may result from oral administration, either from nausea or degradation in the gut or liver.

Another preferred embodiment is oral administration.

### Therapeutically effective amount

The cyclic peptides or the pharmaceutical compositions of the invention are provided such that they comprise a therapeutically effective amount of said cyclic peptide(s) or of said pharmaceutical composition(s).

A "therapeutically effective amount" of a cyclic peptide or a pharmaceutical composition of this invention refers to the amount that is sufficient to induce a reduction of ≥50% in clinical symptoms of the treated disease. Within the context of this invention, this includes but is not exclusively limited, to a reduction of ≥50% in the levels of pain within a patient as determined by the normal clinical parameters.

A preferred therapeutically effective amount is in the range of 10 µg to 1 mg per kg body weight, preferably 10 µg to 100 µg.

The preferred therapeutically effective amount depends on the respective application and desired outcome of inhibition, treatment or prevention.

The skilled artisan will be able to determine suitable therapeutically effective amounts.

### Preferred embodiments

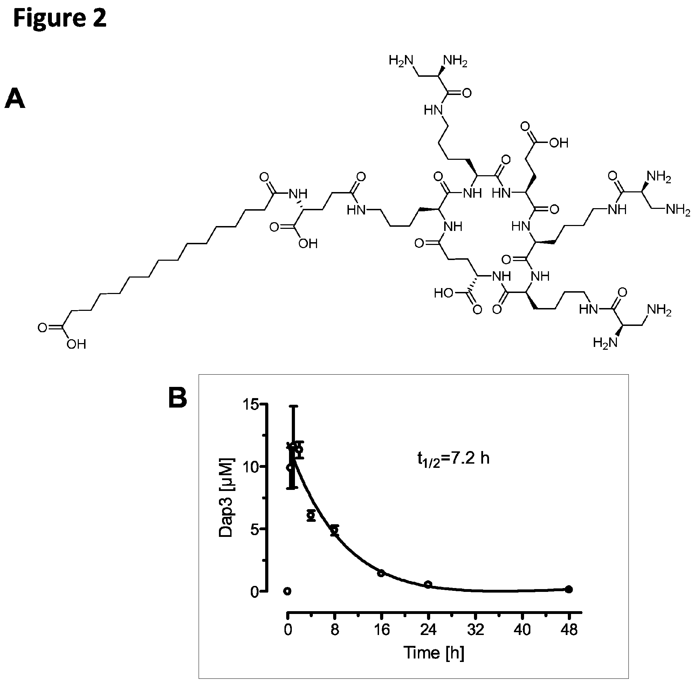

The inventors developed, for example, the MG scavenging peptide Dap3: 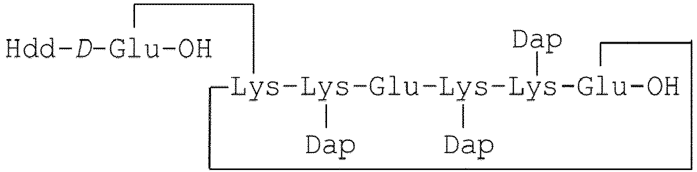 (see also Figure 2A). Dap3 is a cyclic peptide coupled to a hexadecadionic acid via a D-glutamic acid linker. Diaminopropionic acid (Dap) is used as the reactive moiety for MG scavenging. Three of these diaminopropionic acid molecules are attached to the peptide via lysine side chains of the peptide backbone. A total of four carboxy groups from hexadecadionic acid and glutamic acid contribute to an almost balanced charge (+2) of the molecule despite the three diamino groups.

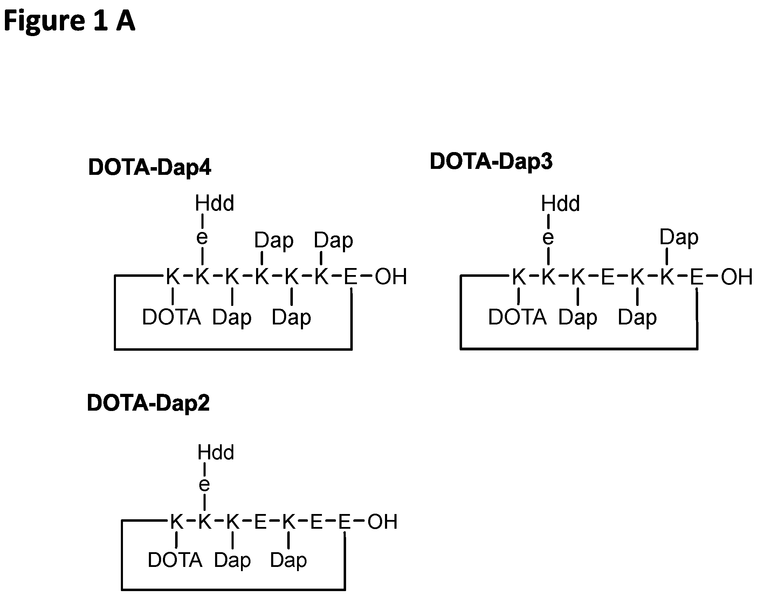

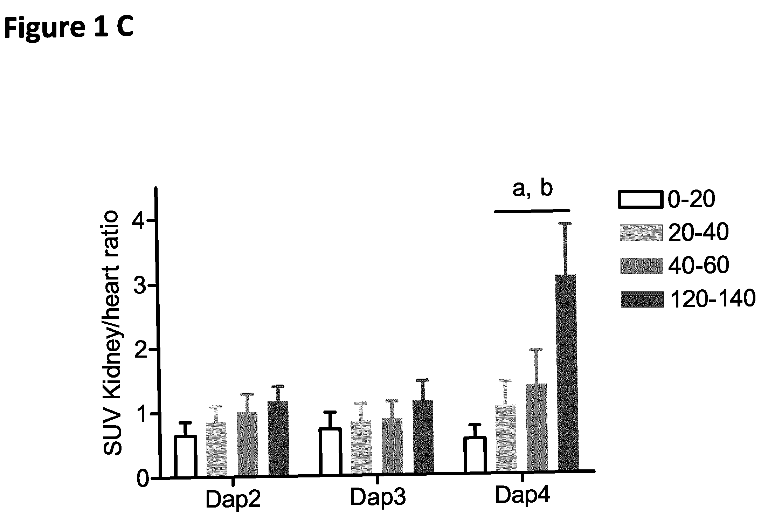

The chelator molecule DOTA (1,4,7,10-Tetraazacyclododecane-1,4,7,10-tetraacetic acid) was coupled to Dap3 and two further cyclic peptides (Dap 2 and Dap 4, see Figure 1A) for labelling with ⁶⁸Ga and the biodistribution of the labelled peptides was compared. Images were obtained after 0-20 min, 20-40 min, 40-60 min and again after 120-140 min, see Figure IB. Starting at 20-40 min it can be seen that kidney uptake is stronger for Dap4 compared to Dap3 and Dap2. This is confirmed by analysing the kidney to heart ratio of the standardized uptake values (Figure 1C) for the measurements between 20-140 min. No difference is seen between Dap3 and Dap2.

Pharmacokinetics of Dap3 was also determined in mice. The scavenger was injected i.p. at 3 µmol/kg and blood was collected at times indicated in Figure 2B. Dap3 was quantified in plasma.

Scavenging activity of the Dap3 towards methylglyoxal was also investigated *in vitro* to compare activity with molecules which were previously reported to scavenge MG. Molecules were incubated at a ratio of 2 reactive sites per molecule MG. Dap3 was amongst the quickest scavenger with activity similar to the known small molecule scavenger aminoguanidine (Table 1).

**Table 1. In vitro MG scavenging of Dap3 in comparison to the small molecule scavenger aminoguanidine and the peptide based scavenger CycK(Myr)R₄E.**

| **Compound** | **MG t_{1/2} [u]** | **95 % CI** |
|---|---|---|
| Dap3 | 0.18 h | 0.15 - 0.24 |
| Aminoguanidine | 0.21 h | 0.17 - 0.27 |
| CycK(Myr)R₄E | 11.33 h | 7.87-20.26 |

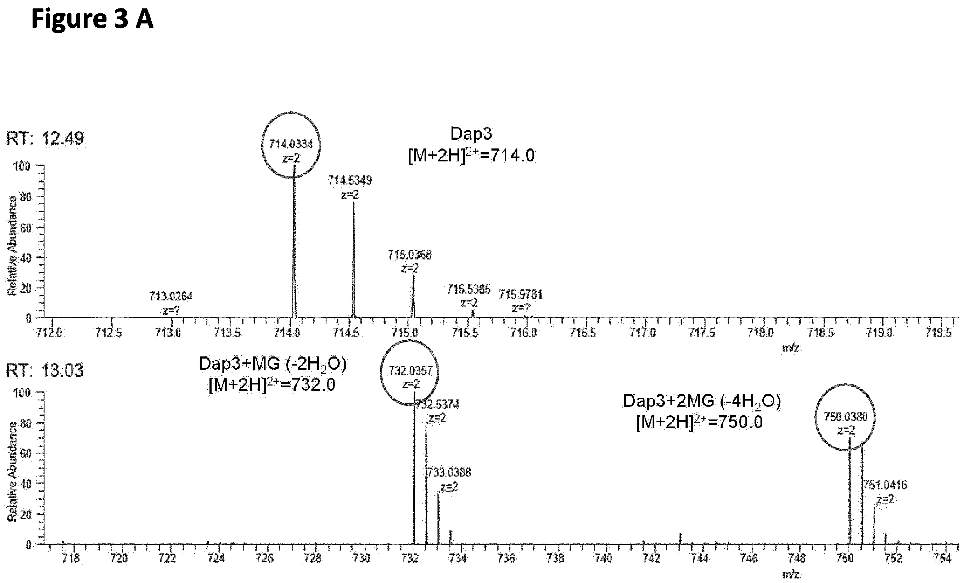

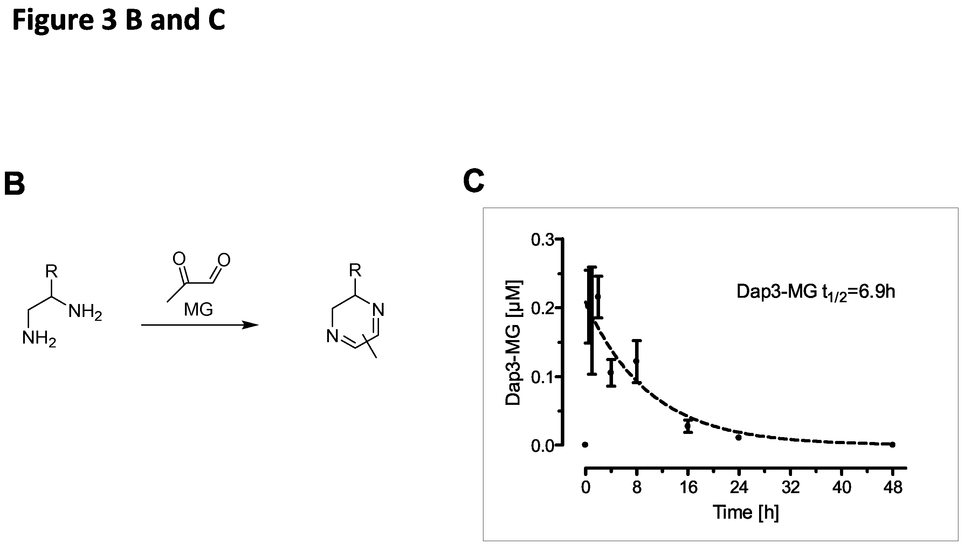

The inventors developed a MG scavenging peptide Dap3 which is highly suitable for the treatment of MG associated diabetic complications. The molecule features a long plasma half-life due to the coupling of hexadecadionic acid and cyclisation of the peptide. Dap3 features three diaminopropionic acid molecules as active sites for MG binding. Thus, the peptides features scavenging activity similar to aminoguanidine, the quickest MG scavenger reported so far. Development of aminoguanidine was stopped due to side effects in clinical trials (Borg *et al.,* 2016). In addition half-life of aminoguanidine is very short (Bowman *et al.,* 1996). The capacity of the scavenger Dap3 to bind MG *in vivo* was proven by the detection of the MG modified peptide in plasma after injection of Dap3 (See Figure 3).

Since peptides are not necessarily being taken up intracellularly it was of interest to determine whether Dap3 is taken up into cells. Thus, we synthesized a propargylglycine modified version of Dap3 which after addition to cells, fixation and permeabilisation, could be modified with a fluorophore in order to determine cellular uptake. The attachment of the fluorophore after the experiment prevents non-specific effects of the fluorophore itself on the cellular uptake.

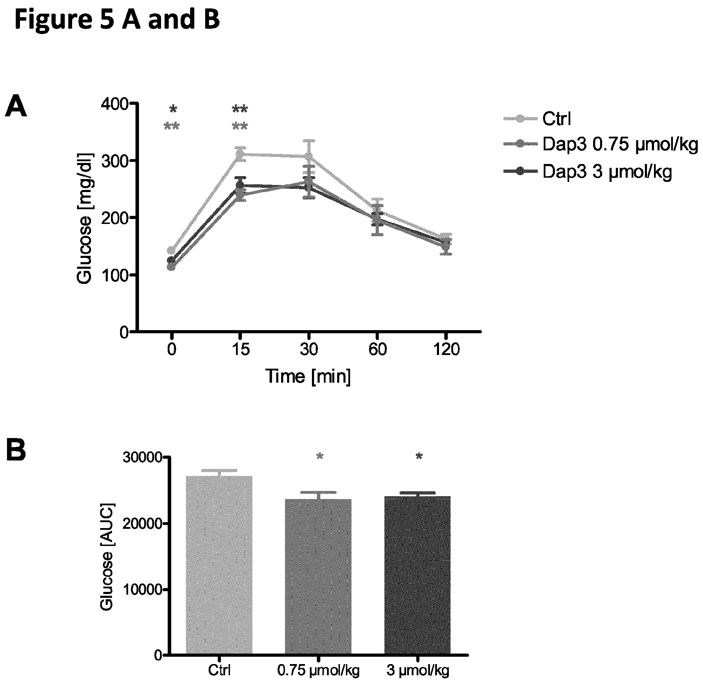

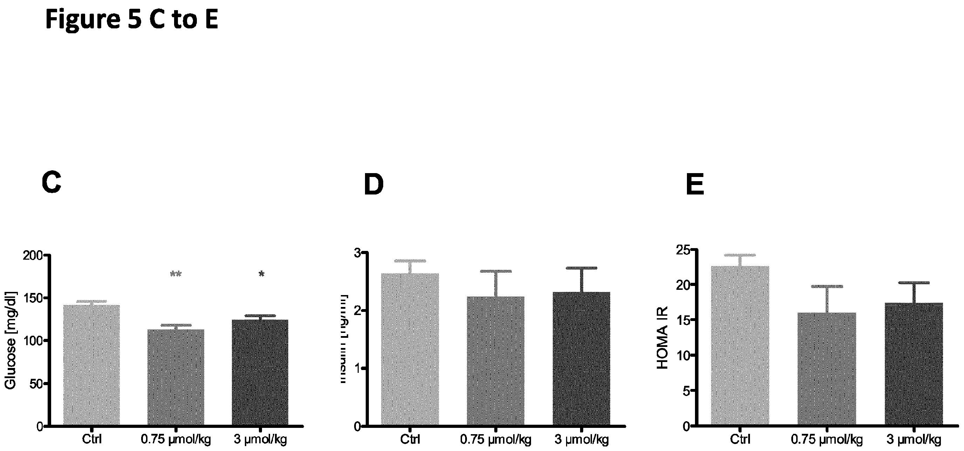

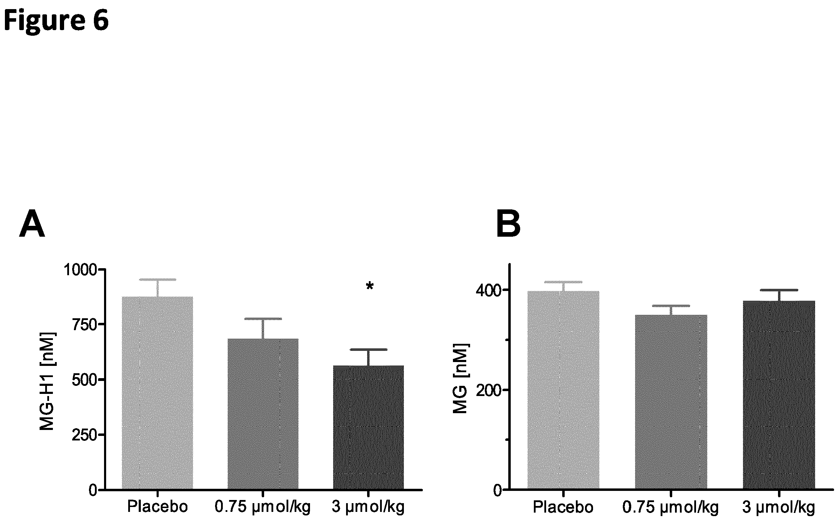

The effect of Dap3 administration on glucose sensitivity in mice with diet-induced obesity is shown in Figure 5. The peptide was administered at daily at two concentrations over 2 weeks and compared to placebo. An intraperitoneal glucose tolerance test carried out at the end of the treatment period displayed improved glucose tolerance at both concentrations (Fig 5 A and B). The improved glucose tolerance was associated with an improved fasting plasma glucose level before administration of the glucose (Fig 5 C) but only a trend for lower insulin levels (Fig 5 D) in combination with a trend for improved insulin resistance was seen (Fig 5 E). In further support of the MG scavenging effect in this setting a dose dependent decrease in MG-H1 levels was seen in a separate study in DIO mice after 4 weeks of treatment with Dap3 (Figure 6).

The following examples and drawings illustrate the present invention without, however, limiting the same thereto.

### BRIEF DESCRIPTION OF THE DRAWINGS

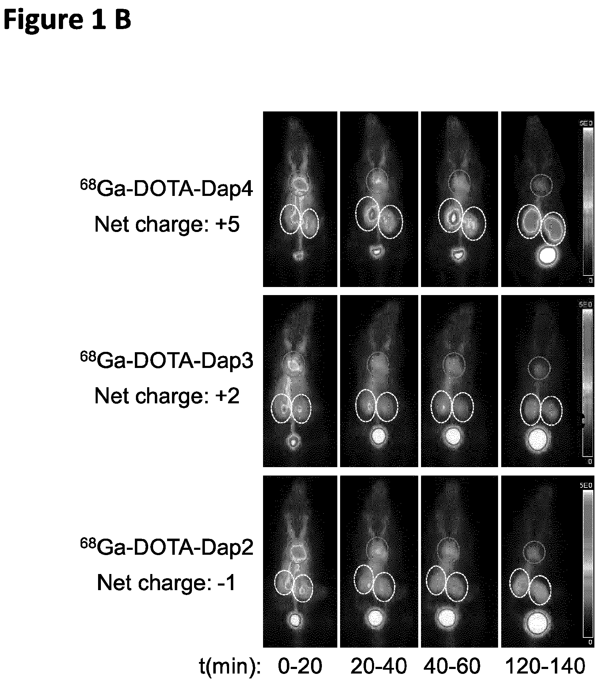

**Figure 1****.** *Biodistribution of the cyclic peptides.*
   Sequence (**A**) and positron emission tomography (PET) of cyclic scavenging peptides (**B**) differing in the amount of diaminopropionic acid are shown One letter code is used for amino acids with uppercase letter for the L-enantiomer and lowercase letter for the D-enantiomer. For PET analysis DOTA-conjugated scavenging peptides were labelled with ⁶⁸Ga prior to i.v. injection and analysis. Images were obtained after 0-20 min, 20-40 min, 40-60 min and again after 120-140 min. Kidneys (white ovals) and heart (red circle) are indicated. Starting at 20-40 min it can be seen that kidney uptake is stronger for Dap4 compared to Dap3 and Dap2. This is confirmed by analysing the kidney to heart ratio of the standardized uptake values (C) for the measurements between 20-140 min (SUV +/- SD; repeated measures ANOVA, a: Dap4 vs. Dap2 p<0.05; b: Dap4 vs. Dap3 p<0.01). The difference in net charge of +2 for Dap3 vs a net charge of -1 for Dap2 is not associated with different biodistribution of these compounds. Since Dap3 contains 3 active sites vs 2 active sites in Dap2, Dap3 is a preferred compound. Non-standard abbreviations are Dap for diaminopropionic acid, Hdd for hexadecanedioic acid and DOTA for the chelator dodecane tetraacetic acid.
**Figure 2****.** *Structure and pharmacokinetics of one cyclic peptide.*
   Molecular structure of Dap3 is shown in (**A**). Hexadecanedioic acid is coupled *via* linker D-glutamic acid to the lysine side chain of the cyclic peptide. Diaminopropionic acid residues are attached to the side chain of three lysines. Pharmacokinetic of Dap3 in mice is shown in (**B**). Dap3 was injected i.p. at 3.0 µmol/kg and blood was collected after indicated times into EDTA containing tubes (B, n=3/time point, +/-SEM). Dap3 was quantified in plasma by LC MS/MS analysis.
**Figure 3****.** *MG scavenging* in vivo.
   Detection of Dap3 MG-adducts *in vitro* and *in vivo.* The scavenging properties of the peptide was tested by incubation with MG *in vitro.* Resulting peptide-MG adducts were analysed by LC MS (**A**). Reaction of methylglyoxal with the active site of diaminopropionic acid (**B**). Analysis of scavenging combined with pharmacokinetic analysis of Dap3-MG adducts *in vivo* was carried out by LC MS/MS employing purified Dap3-MG adducts as standard. Dap3 was injected i.p. at 3.0 µmol/kg and blood was collected at indicated times into EDTA containing tubes (n=3/time point, +/-SEM). The resulting Dap3-MG adducts were quantified in EDTA plasma by LC MS/MS analysis (**C).**

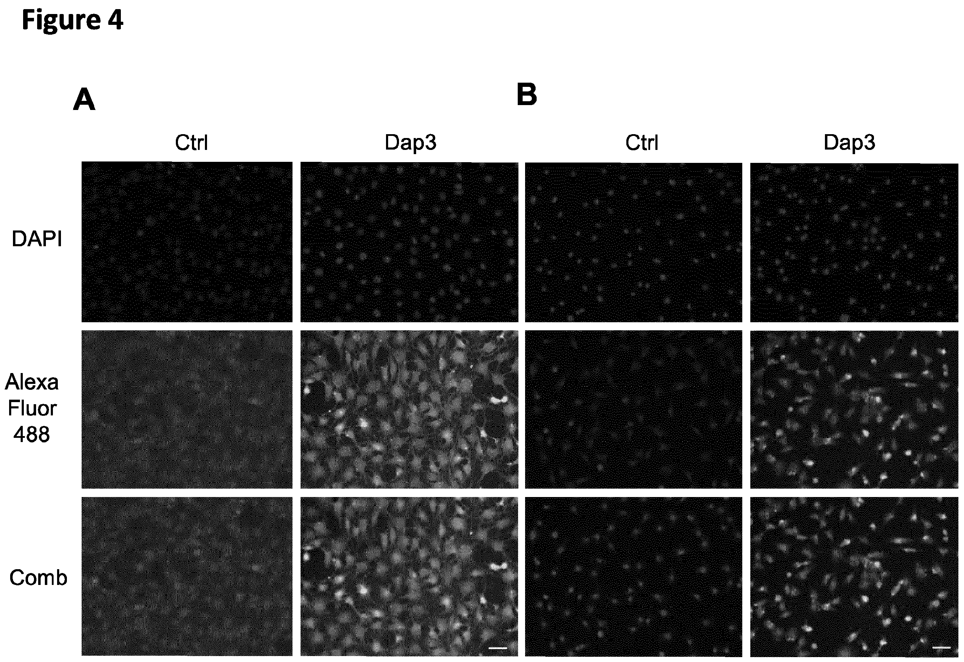

**Figure 4****.** *Intracellular uptake of the cyclic peptide Dap3.*
   Intracellular uptake of Dap3. Propargylglycine conjugated Dap3 was synthesised. Murine embryonic fibroblasts (**A**) and murine cardiac endothelial (**B**) cells were seeded on cover slips, incubated with Dap3 at 500 µM *in vitro* and stained by coupling with azide conjugated AlexaFluor488. Samples were analysed by immunofluorescence microscopy. Scale bar=90 µm.
**Figure 5****.** *Improved glucose tolerance in DIO mice after treatment with the cyclic peptide Dap3.*
   DIO mice were treated with placebo (ctrl) or Dap3 for 2 weeks followed by a glucose tolerance test (GTT). For this 2 g glucose per kg body weight were injected intraperitoneally after a 6 h fast and blood glucose was measured at indicated time points (**A**). Glucose area under the curve was lower in treated animals although no dose dependent effect was seen (**B**). Baseline glucose measurement (**C**) is lower in Dap3 treated animals. Baseline insulin measurement (**D**) and HOMA IR (**E**) tend to be lower in treated animals. Dunnets multiple comparison test (^{∗} p <0.05, ^{∗∗} p<0.01 vs ctrl). No differences between fat mass and lean mass were observed (data not shown). The improved glucose tolerance test after treatment with Dap3 was confirmed in a second experiment. DIO mice were treated for 2 weeks or 4 weeks with the cyclic peptide. A significant improvement of the GTT was seen after 4 weeks of treatment but not yet after 2 weeks of treatment (data not shown).
Figure 6. *Treatment with Dap3 lowers free MG-H1 levels in plasma after 4 weeks of treatment*
   In a second experiment treatment of DIO mice with Dap3 for 4 weeks resulted in an improved intraperitoneal GTT (data not shown). This coincided with lower levels of the methylglyoxal arginine adduct MG-H1 in plasma (**A**). Methylglyoxal plasma levels were not lowered by treatment (**B**). The fact that plasma MG levels do not reflect the decrease in MG-H1 could mean that the origin of the free MG-H1 levels is intracellular rather than extracellular.

### EXAMPLES

### 1. Materials

Protected amino acids were purchased from Orpegen Chemicals. All other materials and chemicals were purchased from Sigma-Aldrich unless indicated otherwise.

### 2. Animal studies

Mice were housed with a 12-hour/12-hour light/dark cycle and had free access to water and food, unless indicated otherwise. All procedures in this study were approved by the Animal Care and Use Committees of the state of Baden-Württemberg or Bavaria, Germany.

### 2.1 Animal studies DIO

C57BL/6-J mice were fed a high fat diet (45 % kcal fat, Research Diets Inc.) until they reached their starting weight for up to 30 weeks. Mice were distributed into experimental groups based on their BWs to assure an equal distribution of BWs at the beginning of the treatment part of the study. Mice were treated with Dap3 or placebo at indicated concentration for 2 weeks (first study) and 2 weeks as well as 4 weeks (second study).

For body composition analysis, fat mass and lean mass were measured via nuclear magnetic resonance technology (EchoMRI). For the GTT, mice were subjected to 6 hours of fasting 1 hour after the onset of the light phase. HFD-fed mice were then injected with 2 g glucose/kg BW i.p. for the GTT. Blood glucose levels (mg/dL) were measured with a handheld glucometer (TheraSense Freestyle) before (0 minutes) and at 15, 30, 60, and 120 minutes after injection.

### 2.2 PET analysis

DOTA coupled peptides were labeled as described in Brings *et al.* (2017). In short, ⁶⁸Ga was eluted from the generator into a tube containing 20 nmol of the peptide and 0.5 % ascorbic acid in 0.5 M Na-acetate buffer. The ⁶⁸Ga-DOTA complex mixture formed upon incubation at pH 3.5-4.0 for 10' at 95 °C while stirring. Free ⁶⁸Ga was removed by solid phase extraction cartridges (SOLA HRP SPE, Thermo Scientific, USA). Quality of the extract was checked by HPLC (Agilent Technologies, USA) equipped with a radio flow detector. PET imaging was performed using a small-animal PET scanner (Inveon; Siemens). The labeled peptide was injected i.v. into Swiss mice. A dynamic scan was carried out for 60 min followed by a 20 min static scan after 2 h. Images were reconstructed and converted to standardized uptake value (SUV) images for 0-20, 20-40, 40-60 and 120-140 min. For quantification of the signal intensity in heart and kidney the SUV in the region-of-interest was also determined.

### 2.3 PK analysis

Animals were injected with Dap3 and blood was collected into EDTA containing tubes at indicated time points with n=3 per time point. After centrifugation EDTA plasma was stored at -80 °C until analysis. Dap3 was quantified by LC MS/MS as described in section 6.

### 3. MG scavenging in vitro

Scavengers were incubated with MG (200 µM) in 0.1 M phosphate buffer, pH 7.4 at 37 °C. Scavenger was added at a concentration to reach 400 µM of active sites. Dap3 was added at 133 µM, CycK(Myr)R₄E was added at 100 µM while aminoguanidine was added at 400 µM. Aliquots were taken after 0.5 h, 1 h, 4 h, 8 h, 24 h and 48 h frozen in liquid nitrogen and MG content was quantified and stored at -80 °C until analysis. MG content was quantified by HPLC as described below.

### 4. Peptide synthesis

Peptides were synthesized on solid phase, using Fmoc-chemistry. Coupling of amino acids (10 eq.) was carried out with HBTU (9.8 eq.) and DIPEA (20 eq.) in NMP and Fmoc was removed by incubation in 20 % piperidine/NMP unless noted otherwise. After coupling and deprotection, resin was washed with NMP. CycK(Myr)R₄E was synthesised as described previously, in Brings *et al.* (2017).

Diaminopropionic acid derivatives were synthesised on Wang resin. Resin was loaded with Fmoc-E(OAll)-OH (4eq.) using triphenylphosphine (4 eq.) and diisopropyl azodicarboxylate (4 eq.) in THF and reaction is left to proceed for 2 h. The reaction was repeated once. The resin was washed with THF and NMP and Fmoc was removed. The four following amino acids Fmoc-K(MTT)-OH or Fmoc-E(tBu)-OH were coupled to reflect the desired sequence (Dap2, -KEKE-; Dap3, -KEKK-; Dap4, -KKKK-). Fmoc was not removed after the last coupling step.

For the DOTA containing peptides MTT was removed by incubation with 2.5 % TIS in DCM for 2 h followed by washes with DCM and NMP. Boc-Dap(Boc)-OH (2 eq.) was coupled to the side chains with HBTU (2 eq.) and DIPEA (4 eq.). Fmoc was removed followed by coupling of Fmoc-K(MTT)-OH and Alloc-K(Fmoc)-OH. Fmoc was removed and Tris-tBu-DOTA (5 eq.) was coupled to the side chain with COMU (5 eq.) and DIPEA (10 eq.) in NMP for 2 h. Next Alloc and OAll protecting groups were removed by incubation with tetrakistriphenylphosphine palladium (5 mg on 250 mg resin equivalent to 50 µmol peptide) and dimethylaminoboran (20 mg for 250 mg resin equivalent to 50 µmol peptid) in DCM for 20 min followed by washes with DCM, methanol and NMP. The peptide was cyclised with PyAOP (5 eq.) and DIPEA (7.5 eq.) in NMP for 1h. MTT was removed as described above and tBu-hexadecanedioic acid (2 eq.) was coupled with HBTU (2 eq.) and DIPEA (4 eq.) in NMP for 2h twice.

Due to the presence of several tBu protecting groups peptides were de-protected and cleaved from the resin with 90 % TFA, 5 % para- Kresol, 2.5 % TIS and 2.5 % water.

For Dap3 synthesis Alloc-K(Fmoc)-OH was coupled after the third Fmoc-K(MTT)-OH followed by Fmoc removal and Fmoc-e(tBu). After Fmoc removal tBu-hexadecanedioic acid was coupled. Alloc and OAll were removed, cyclisation was carried out as described above. For propargylglycine containing Dap3 Fmoc-propargyl-Gly-OH was added after the third Fmoc-K(MTT)-OH and before coupling of Alloc-K(Fmoc)-OH. The peptides were de-protected and cleaved from the resin with 2.5 % TIS, 2.5 % water in TFA for 3h followed by precipitation in diethylether.

All peptides were purified by preparative revers phase HPLC with solvent A being water 0.1 % TFA and acetonitrile 0.1 % TFA as solvent B. LC MS analysis of peptides was carried out on Exactive Orbitrap instrument (Thermo Scientific, USA).

### 5. MG quantification by HPLC and LC MS/MS

MG was quantified as described previously (Brings *et al.,* 2017; Rabbini *et al.,* 2014). HPLC based methods were employed for the quantification *in vitro* while LC MS/MS based methods were used for the quantification *in vivo.* Proteins were precipitated by addition of 20 % TCA at a ratio of 2/1 followed by vortexing. Internal standards were added and the sample mixed again. Samples were centrifuged, and the supernatant derivatised with 1,2 diaminobenzene. For MG analysis by HPLC water + 0.1 % TFA was used as solvent A and solvent B was acetonitrile +0.1 % TFA. A linear gradient from 0-6 % solvent B at a flow rate of 2 ml/min over 15 min was used. The MG-1,2-diaminobenzene adduct 2-methylquinoxaline and the internal standard 2,3-dimethylquinoxaline were detected with a UV monitor. For LC-MS/MS based analysis, samples were run on an ACQUITY UPLC I equipped with a Xevo TQ-XS mass spectrometer (Waters, USA). Samples were separated on BEH C18 analytical column (2.1 × 50mm, 1.7µm) fitted with pre-column (2.1 × 5mm, 1.7µm). For LC MS/MS analysis solvent A was water +0.1% formic and solvent B was 50% acetonitrile/water +0.1% formic acid. A linear gradient from 0-100% solvent B, over ten minutes was used with a flow rate of 0.2ml/min. The quinoxaline analytes were detected by electrospray positive ionization-mass spectrometric multiple-reaction monitoring (MRM).

### 6. Detection of Dap3 and MG-modified Dap3 by LC-MS/MS

A Dap3-MG standard was prepared by incubation of Dap3 with methylglyoxal in 0.1 M phosphate buffer followed by purification by preparative HPLC. The peptide CycK(e-Hdd)R₄E was employed as internal standard which was added to the standard curve and plasma samples prior to precipitation. Prior to analysis 2 parts acetonitrile were added to the plasma, vortexed and centrifuged for 10 min at 13,000 g. The supernatant was analysed using an ACQUITY UPLC I fitted with a Acquity UPLC BEH C18 column (Waters, 2.1 mm ^{∗} 100 mm, 17 µm) with a pre-column (2.1 × 5mm, 1.7µm) and a Xevo TQ-XS mass spectrometer (Waters, USA). Solvent A was water + 0.1 % formic acid and solvent B was acetonitrile + 0.1% formic acid. A linear gradient from 0-100 % solvent B at a flow rate of 0.2 ml/min over 20 min and a column temperature of 30 °C was used. The detailed parameters for the quantification of Dap3, Dap3-MG and CycK(e-Hdd)R₄E are described below.

**Table 2.**

| **Molecule** | **Type** | **Mass transitions** | **Collision energy [eV]** | **Retention time [min]** |
|---|---|---|---|---|
| Dap3 | Quantifier | 476.44 > 505.47 | 20.0 | 4.78 |
| | Qualifier | 714.16 > 705.24 | 26.0 | 4.78 |
| Dap3-MG | Quantifier | 488.54 > 533.50 | 20.0 | 5.45 |
| | Qualifier | 488.52 > 378.02 | 22.0 | 5.46 |
| CycK(e-Hdd)R₄E | Quantifier | 427.39 > 84.04 | 34.0 | 5.35 |
| | Qualifier | 427.39 > 70.05 | 54.0 | 5.35 |

### 7. Intracellular uptake of Dap3

The intracellular uptake of Dap3 was assessed *in vitro* by fluorescence microscopy. Immortalised murine cardiac endothelial cells (MCEC) and murine embryonic fibroblasts (MEF) were grown under standard growth conditions in DMEM supplemented with FCS at 37°C with 5%CO₂. For MCEC all surfaces including cover slips were coated with 0.5 % gelatine/PBS prior to seeding of the cells. For immunofluorescence microscopy cells were seeded onto 3 well chamber slides at a density of 5×10⁴ cells per chamber in 500 µl. Cells were incubated with 500 µM propargylglycine modified Dap3 or medium only as control overnight. Cells were washed and fixated with 4 % PFA the next day followed by permeabilisation with 0.1 % Triton X100 in FACS buffer. After washing of cells, Alexa Fluor 488 Azide (C10327, Thermo Fisher Scientific) and Click-it cocktail (C10269, Thermo Fisher Scientific) were added. Cells were washed with 0.1 % Triton X100 in FACS buffer and mounted with DAPI containing mounting medium (HP20.1, Roth). Cells were analysed by immunofluorescence microscopy and images were processed using Image J.

The features disclosed in the foregoing description, in the claims and/or in the accompanying drawings may, both separately and in any combination thereof, be material for realizing the invention in diverse forms thereof.

### REFERENCES

F. M. Abu-Zidan, L. D. Plank and J. A. Windsor, Eur J Surg 2002, 168, 119-123.

S. Agalou, N. Ahmed, A. Dawnay and P. J. Thornalley, Biochem Soc Trans 2003, 31, 1394-1396.

N. Ahmed, U. Ahmed, P. J. Thornalley, K. Hager, G. Fleischer and G. Munch, J Neurochem 2005, 92, 255-263.

M. Aksenov, M. Aksenova, D. A. Butterfield and W. R. Markesbery, J Neurochem 2000, 74, 2520-2527.

M. Y. Aksenov, M. V. Aksenova, D. A. Butterfield, J. W. Geddes and W. R. Markesbery, Neuroscience 2001, 103, 373-383.

P. J. Beisswenger, K. S. Drummond, R. G. Nelson, S. K. Howell, B. S. Szwergold, M. Mauer, Diabetes 2005, 54, 3274-3281.

P.J. Beisswenger, S. K. Howell, G. B. Russell, M. E. Miller, S. S. Rich, M. Mauer, Diabetes Care 2013, 36, 3234-3239.

A. Bierhaus, T. Fleming, S. Stoyanov, A. Leffler, A. Babes, C. Neacsu, S. K. Sauer, M. Eberhardt, M. Schnolzer, F. Lasitschka, W. L. Neuhuber, T. I. Kichko, I. Konrade, R. Elvert, W. Mier, V. Pirags, I. K. Lukic, M. Morcos, T. Dehmer, N. Rabbani, P. J. Thornalley, D. Edelstein, C. Nau, J. Forbes, P. M. Humpert, M. Schwaninger, D. Ziegler, D. M. Stern, M. E. Cooper, U. Haberkorn, M. Brownlee, P. W. Reeh, P. P. Nawroth, Nature medicine 2012, 18, 926-933.

Borg, D. J., and Forbes, J. M. (2016) Targeting advanced glycation with pharmaceutical agents: where are we now? Glycoconjugate journal 33, 653-670.

F. Boscia, I. Grattagliano, G. Vendemiale, T. Micelli-Ferrari and E. Altomare, Invest Ophthalmol Vis Sci 2000, 41, 2461-2465.

Bowman, M. A., Simell, O. G., Peck, A. B., Cornelius, J., Luchetta, R., Look, Z., Maclaren, N. K., and Atkinson, M. A. (1996) Pharmacokinetics of aminoguanidine administration and effects on the diabetes frequency in nonobese diabetic mice. The journal of pharmacology and experimental therapeutics 279, 790-794.

Brings, S., Fleming, T., De Buhr, S., Beijer, B., Lindner, T., Wischnjow, A., Kender, Z., Peters, V., Kopf, S., Haberkorn, U., Mier, W., and Nawroth, P. P. (2017) A scavenger peptide prevents methylglyoxal induced pain in mice. Biochimica et biophysica acta 1863, 654-662.

D. A. Butterfield and C. M. Lauderback, Free Radic Biol Med 2002, 32, 1050-1060.

A. Castegna, M. Aksenov, V. Thongboonkerd, J. B. Klein, W. M. Pierce, R. Booze, W. R. Markesbery and D. A. Butterfield, J Neurochem 2002, 82, 1524-1532.

K. Chen, M. Kazachkov and P. H. Yu, J Neural Transm (Vienna) 2007, 114, 835-839.

J. Choi, C. A. Malakowsky, J. M. Talent, C. C. Conrad and R. W. Gracy, Biochem Biophys Res Commun 2002, 293, 1566-1570.

C. C. Conrad, P. L. Marshall, J. M. Talent, C. A. Malakowsky, J. Choi and R. W. Gracy, Biochem Biophys Res Commun 2000, 275, 678-681.

S. Dimon-Gadal, P. Gerbaud, P. Therond, J. Guibourdenche, W. B. Anderson, D. Evain-Brion and F. Raynaud, J Invest Dermatol 2000, 114, 984-989.

I. Dhar, A. Dhar, L. Wu, K. Desai, J. pharmacology and experimental therapeutics 2012, 342(1), 196-204.

L. Engelen, C. D. Stehouwer, C. G. Schalkwijk, Diabetes, obesity & metabolism 2013, 15, 677-689.

R. J. Ferrante, S. E. Browne, L. A. Shinobu, A. C. Bowling, M. J. Baik, U. MacGarvey, N. W. Kowall, R. H. Brown, Jr. and M. F. Beal, J Neurochem 1997, 69, 2064-2074.

T. Fleming, J. Cuny, G. Nawroth, Z. Djuric, P. M. Humpert, M. Zeier, A. Bierhaus, P. P. Nawroth, Diabetologia 2012, 55, 1151-1155.

E. Floor and M. G. Wetzel, J Neurochem 1998, 70, 268-275.

J. M. Forbes, M. E. Cooper, Physiol Rev 2013, 93, 137-188.

S. Genuth, W. Sun, P. Cleary, X. Gao, D. R. Sell, J. Lachin, D. E. R. Group, V. M. Monnier. Diabetes 2015, 64, 266-278.

F. Giacco, X. Du, V. D. D'Agati, R. Milne, G. Sui, M. Geoffrion and M. Brownlee, Diabetes 2014, 63, 291-299.

K. Hensley, N. Hall, R. Subramaniam, P. Cole, M. Harris, M. Aksenov, M. Aksenova, S. P. Gabbita, J. F. Wu, J. M. Carney and et al., J Neurochem 1995, 65, 2146-2156.

J. Himmelfarb and E. McMonagle, Kidney Int 2001, 60, 358-363.

J. Himmelfarb, E. McMonagle and E. McMenamin, Kidney Int 2000, 58, 2571-2578.

A. Lapolla, R. Flamini, A. Lupo, N. C. Arico, C. Rugiu, R. Reitano, M. Tubaro, E. Ragazzi, R. Seraglia and P. Traldi, Ann N YAcad Sci 2005, 1043, 217-224.

P. S. Lim, Y. M. Cheng and Y. H. Wei, Free Radic Res 2002, 36, 295-301.

T. W. Lo, T. Selwood, P. J. Thornalley, Biochemical pharmacology 1994, 48, 1865-1870.

T. W. Lo, M. E. Westwood, A. C. McLellan, T. Selwood, P. J. Thornalley, The Journal of biological chemistry 1994, 269, 32299-32305.

L. Lyras, R. H. Perry, E. K. Perry, P. G. Ince, A. Jenner, P. Jenner and B. Halliwell, J Neurochem 1998, 71, 302-312.

D. E. Maessen, C. D. Stehouwer and C. G. Schalkwijk, Clin Sci (Lond) 2015, 128, 839-861.

D. Mantle, G. Falkous and D. Walker, Clin Chim Acta 1999, 284, 45-58.

L. T. McGrath, P. Mallon, L. Dowey, B. Silke, E. McClean, M. McDonnell, A. Devine, S. Copeland and S. Elborn, Thorax 1999, 54, 518-523.

T. Miyata, C. van Ypersele de Strihou, K. Kurokawa and J. W. Baynes, Kidney Int 1999, 55, 389-399.

T. Miyata, Y. Ueda, A. Saito and K. Kurokawa, Nephrol Dial Transplant 2000, 15 Suppl 1, 25-28.

S. Muller-Krebs, L. P. Kihm, B. Zeier, M. L. Gross, R. Deppisch, A. Wieslander, T. Henle, I. Penndorf, J. Oh, J. Reiser, P. P. Nawroth, M. Zeier and V. Schwenger, Eur J Clin Invest 2008, 38, 296-305.

G. Munch, B. Kuhla, H. J. Luth, T. Arendt and S. R. Robinson, Biochem Soc Trans 2003, 31, 1397-1399.

K. Nakayama, M. Nakayama, M. Iwabuchi, H. Terawaki, T. Sato, M. Kohno and S. Ito, Am J Nephrol 2008, 28, 871-878.

U. Pantke, T. Volk, M. Schmutzler, W. J. Kox, N. Sitte and T. Grune, Free Radic Biol Med 1999, 27, 1080-1086.

N. Rabbani, K. Sebekova, K. Sebekova, Jr., A. Heidland and P. J. Thornalley, Kidney Int 2007, 72, 1113-1121.

N. Rabbani, P. J. Thornalley, Nature protocols 2014, 9, 1969-1979.

N. Rabbani and P. J. Thornalley, Biochemical and Biophysical Research Communications 2015, 458, 221-226.

N. Rabbani, M. V. Chittari, C. W. Bodmer, D. Zehnder, A. Ceriello and P. J. Thornalley, Diabetes 2010 59, 1038-1045.

N. Rabbani, L. Godfrey, M. Xue, F. Shaheen, M. Geoffrion, R. Milne and P. J. Thornalley, Diabetes 2011 60, 1973-1980.

S. P. Range, C. Dunster, A. J. Knox and F. J. Kelly, Eur Respir J 1999, 13, 560-564.

J. Renke, S. Popadiuk, M. Korzon, B. Bugajczyk and M. Wozniak, Free Radic Biol Med 2000, 29, 101-104.

Schalkwijk, C. G., M. A. Vermeer, C. D. Stehouwer, J. te Koppele, H. M. Princen and V. W. van Hinsbergh, Biochim Biophys Acta, 1998 1394, 187-198.

F. A. Shamsi, K. Lin, C. Sady and R. H. Nagaraj, Invest Ophthalmol Vis Sci 1998, 39, 2355-2364.

K. Shinpo, S. Kikuchi, H. Sasaki, A. Ogata, F. Moriwaka and K. Tashiro, Brain Res 2000, 861, 151-159.

P. J. Thornalley, S. Battah, N. Ahmed, N. Karachalias, S. Agalou, R. Babaei-Jadidi, A. Dawnay, The Biochemical journal 2003, 375, 581-592.

C. C. Winterbourn, I. H. Buss, T. P. Chan, L. D. Plank, M. A. Clark and J. A. Windsor, Crit Care Med 2000, 28, 143-149.

G. Yang, G. I. Cancino, S. K. Zahr, A. Guskjolen, A. Voronova, D. Gallagher, P. W. Frankland, D. R. Kaplan and F. D. Miller, Cell Reports 2016, 17, 1022-1036.

P. L. Zusterzeel, H. Rutten, H. M. Roelofs, W. H. Peters and E. A. Steegers, Placenta 2001, 22, 213-219.

## Claims

1. A cyclic peptide,
which has a length of 3 to 12 amino acids and comprises
(i) at least two **Lys**,
(ii) at least one **amino acid with an acidic side chain,** preferably Glu or Asp,
(ii) at least one **Dap** (2,3-diaminopropanoic acid) attached to the side chain of Lys,
and
(iii) at least one hydrophobic modification **H.**

2. The cyclic peptide according to claim 1, which is cyclized via
(a) head-to-side chain cyclization, preferably the side chain of the C-terminal Glu,
(b) head-to-tail cyclization,
(c) backbone cyclization,
(d) amide condensation of two amino acid side chains (lactam),
(e) thioether formation,
(f) hydrogen bond formation,
and/or
(g) side chain-to-tail cyclization,
preferably (a).

3. The cyclic peptide according to claim 1 or 2, which is **characterized by** one or more of the following:
- a length of 5 to 10 amino acids, preferably 6 to 8 amino acids, such as 6 or 7 amino acids,
- a net charge of about ≤+5,
- comprising 2 to 6 Lys, preferably 3 or 4 or 5 Lys,
- comprising 1 to 3 amino acids with an acidic side chain, preferably 1 to 3 Glu and/or Asp, such as 2 Glu,
- comprising 1 to 4 Dap, preferably 2 to 4 Dap, more preferably 2 or 3 Dap,
and/or
- having a plasma half-life of up to about one week.

4. The cyclic peptide according to any of claims 1 to 3, wherein the hydrophobic modification **H** is an acylation,
preferably an acylation with C10 to C22 fatty acids, more preferably C12 to C22 fatty acids, such as myristoyl (C14), palmitoyl (C16) or stearoyl (C18), more preferably myristoyl (C14), or an acylation with C10 to C22 dicarboxylic acids, more preferably C12 to C22 dicarboxylic acids, such as tetradecanedioic acid (C14), hexadecanedioic acid (C16) (Hdd), octadecanedioic acid (C18), more preferably hexadecanedioic acid (C16),
or an acylation with a fatty acid containing phenyl group, such as 4-(*p*-iodophenyl)butyric acid.

5. The cyclic peptide according to any of claims 1 to 4, wherein the hydrophobic modification is attached to the side chain of a lysine residue close to the or at the N-terminus, optionally via a linker,
and/or wherein the linker is an amino acid, preferably at least one *D*-Glu, preferably one *D*-Glu.

6. The cyclic peptide according to any of claims 1 to 5, comprising an amino acid sequence selected from
Lys - Lys - Lys - Lys - Lys - Glu [SEQ ID NO. 1],
Lys - Lys - Glu - Lys - Lys - Glu [SEQ ID NO. 2],
Lys - Lys - Glu - Lys - Glu - Glu [SEQ ID NO. 3], and
Lys - Glu - Lys - Glu - Lys - Lys - Glu [SEQ ID NO. 4].

7. The cyclic peptide according to any of claims 1 to 6 being selected from 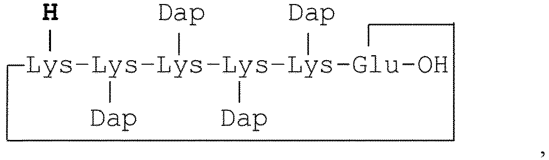 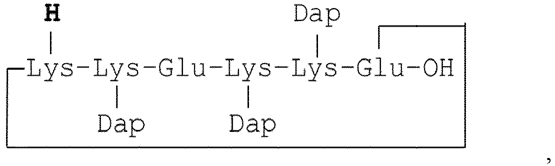 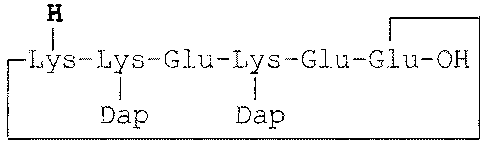 , or 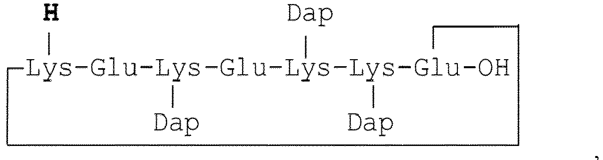 preferably

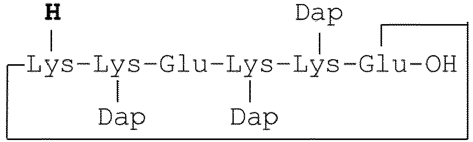

8. The cyclic peptide according to any of claims 1 to 7 being selected from 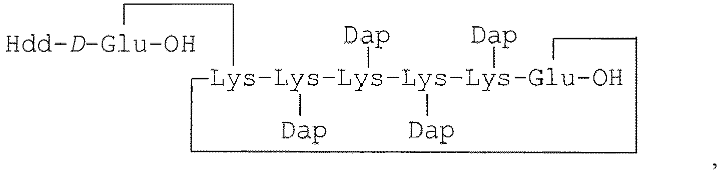 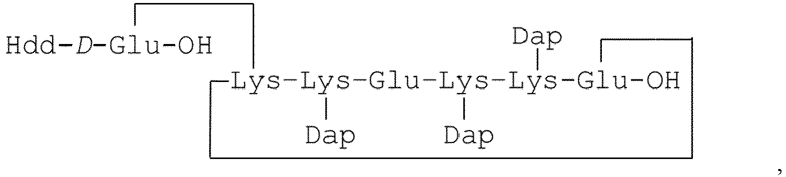 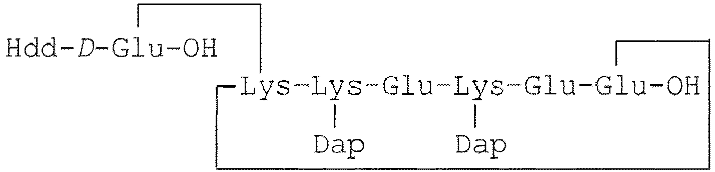 , or 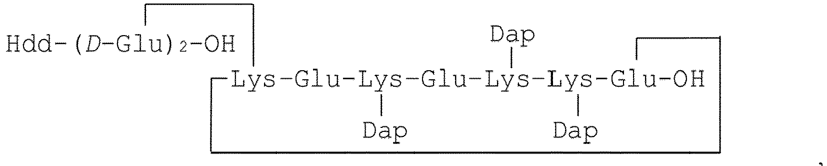 preferably

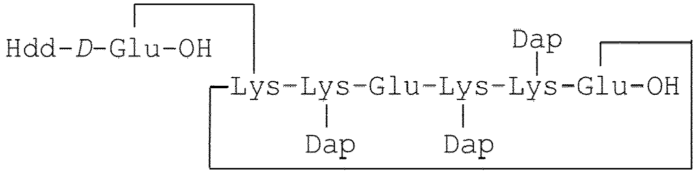

9. The cyclic peptide according to any of the foregoing claims, comprising one or more further compounds, such as tags or labels,
and/or which inhibits the binding of methylglyoxal (MG) and/or reactive carbonyl species (RCS) to an arginine- or lysine-containing protein.

10. The cyclic peptide according to any of claims 1 to 9 for use in medicine.

11. The cyclic peptide according to any of claims 1 to 9 suitable for use as scavenger of methylglyoxal and/or reactive carbonyl species (RCS).

12. The cyclic peptide according to any of claims 1 to 9 suitable for use as antagonist for binding to arginine-containing protein(s), preferably an arginine containing extracellular or intracellular protein, such as
- low density lipoprotein,
- Nav1.8,
- glomerular and tubular proteins of the kidney.

13. The cyclic peptide according to any of claims 1 to 9 for use in a method of prevention and/or treatment of a disease caused by or associated with methylglyoxal (MG) and/or reactive carbonyl species (RCS), in particular caused by or associated with elevated MG levels.

14. The cyclic peptide for use according to claim 13, wherein the disease caused by or associated with methylglyoxal (MG) and/or reactive carbonyl species (RCS), in particular caused by or associated with elevated MG levels is selected from:
- diabetes and its associated complications,
wherein said associated complications comprise diabetic neuropathy (such as pain and/or hyperalgesia), diabetic nephropathy (such as albuminuria and/or lowered eGFR), diabetic retinopathy and endothelial dysfunction,
- cardiovascular disease,
in particular atherosclerosis,
- obesity,
- Alzheimers disease, amyotrophic lateral sclerosis, cataractogenesis, chronic renal failure and chronic or acute Uraemia, cystic fibrosis, dementia with Lewy bodies, ischaemia-reperfusion, pre-eclampsia, psoriasis, rheumatoid arthritis and juvenile chronic arthritis, severe sepsis, systemic amyloidosis and Parkinson's disease.

15. A pharmaceutical composition comprising
at least one cyclic peptide according to any of claims 1 to 9,
optionally a pharmaceutically acceptable carrier and/or excipient.
